# EUROPÄISCHE PATENTSCHRIFT

(11) **EP 2 349 041 B1**
(45) Veröffentlichungstag und Bekanntmachung des Hinweises auf die Patenterteilung: **25.04.2018**
(21) Anmeldenummer: 09777786.6
(22) Anmeldetag: 10.08.2009
(51) Int. Cl.: A61B 18/00, A61B 18/12, H03L 5/00

(54) **HF-CHIRURGIEGENERATOR**
HF SURGICAL GENERATOR
GÉNÉRATEUR CHIRURGICAL HF

(30) Priorität: 08.09.2008 DE 102008046247; 24.11.2008 DE 102008058737
(43) Veröffentlichungstag der Anmeldung: 03.08.2011
(73) Patentinhaber: Erbe Elektromedizin GmbH, 72072 Tübingen (DE)
(72) Erfinder: SCHALL, Heiko, 72622 Nürtingen (DE); EISELE, Florian, 79108 Freiburg (DE)
(74) Vertreter: Bohnenberger, Johannes
(86) Internationale Anmeldenummer: PCT/EP2009/005797
(87) Internationale Veröffentlichungsnummer: WO 2010/025807

(56) Entgegenhaltungen:
- EP-A- 1 543 788
- US-A- 5 304 214
- US-A- 5 836 943
- US-A1- 2002 165 530
- US-A1- 2005 143 725
- DATABASE WPI Week 200781 Thomson Scientific, London, GB; AN 2007-875007 XP002551484 & CN 1 983 785 A (CHINA SCI INST ELECTRICAL RES) 20. Juni 2007 (2007-06-20)

## Beschreibung

Die Erfindung betrifft einen HF-Chirurgiegenerator nach dem Oberbegriff des Patentanspruches 1 sowie ein Verfahren zum Erzeugen einer Hochfrequenzspannung in einem HF-Chirurgiegenerator nach Anspruch 10. In der Chirurgie werden in zunehmendem Maße HF-chirurgische Geräte verwendet. Die hierfür verwendeten Generatoren liefern eine Grundfrequenz, die typischerweise zwischen 300 kHz und 4 MHz beträgt. In den Generatoren sind Leistungsoszillatoren vorgesehen, welche eine aus dem Energieversorgungsnetz bereitgestellte und gleichgerichtete elektrische Energie in eine potentialfreie, gleichspannungsfreie Ausgangsspannung mit der genannten Grundfrequenz umwandeln. Dieser Grundfrequenz wird in sehr vielen Anwendungsfällen, z.B. beim Hochspannungs-Koagulieren oder beim koagulierenden Schneiden eine so genannte Modulationsfrequenz überlagert, die typischerweise in der Größenordnung bis 50 kHz liegt. Dabei soll lediglich eine begrenzte Anzahl von Sinusschwingungen, im Extremfall eine einzige Sinusschwingung erzeugt werden, woran sich eine Pulspause ohne Energieübertragung anschließt. Nach Ablauf einer Modulationsperiodendauer beginnt wieder das Zur-Verfügung-Stellen eines Pulspaketes.

Es ist hieraus ersichtlich, dass ein spontanes Anschwingen der Ausgangsspannung gefordert wird. Dies bedeutet, dass nach 1, spätestens 2 Halbperioden, die Ausgangsspannung ihren Endwert erreicht haben muss, da dies den angestrebten Effekt auf das behandelte Gewebe beeinflusst.

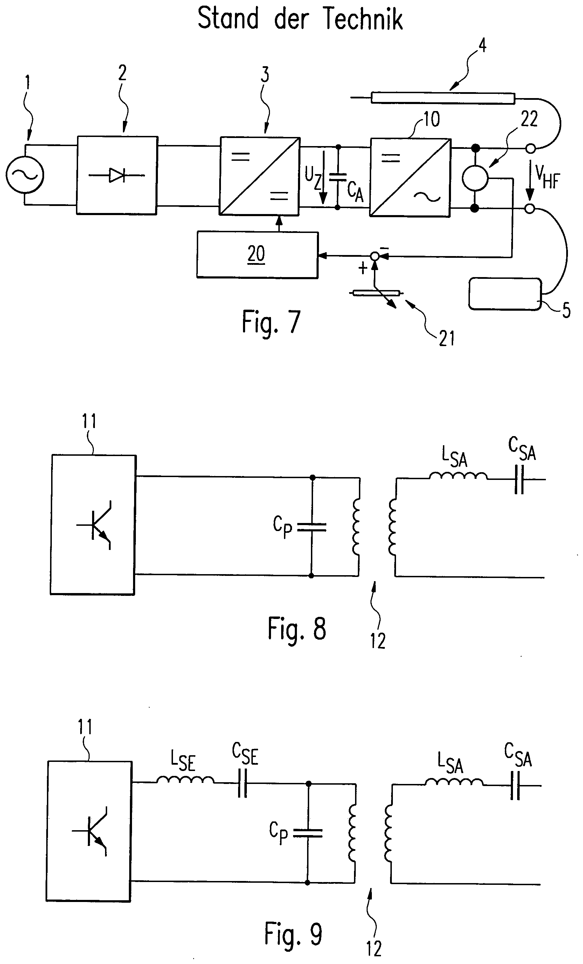

Üblicherweise wird die beschriebene Energiewandlung in zwei aufeinander folgenden Schritten bewerkstelligt, wie dies in Fig. 7 gezeigt ist. Zunächst wird die vom Netz 1 gelieferte elektrische Energie durch einen Gleichrichter 2 gleichgerichtet, so dass eine konstante Gleichspannung zur Verfügung steht. Diese konstante Gleichspannung wird durch ein Netzgerät 3 (einen DC/DC-Wandler) in eine so genannte Zwischenkreisspannung U_{Z} umgewandelt. Diese Zwischenkreisspannung U_{Z} ist einstellbar. Diese erste Energiewandlung wird im Folgenden als Netzgerät bezeichnet.

Daran schließt sich eine zweite Energiewandlung an, die im Folgenden als Leistungsoszillator 10 bezeichnet wird. Der Leistungsoszillator 10 ist dabei ein Wechselrichter, der im Allgemeinen auch eine Potentialtrennung zum Patientenkreis beinhaltet. Die Ausgangsklemmen des Leistungsoszillators 10 sind einerseits an ein elektrochirurgisches Instrument 4 und andererseits an eine Neutralelektrode 5 angeschlossen. Weiterhin liegt an den Ausgangsklemmen des Leistungsoszillators 10 ein Istwertgeber 22, der die Spannung am Ausgang des Leistungsoszillators 10 abtastet und mit einer Sollspannung aus einem Sollwertgeber 21 vergleicht, woraufhin die Regelabweichung über einen Regler 20 zum Netzgerät 3 zurückgeführt wird, so dass die Zwischenkreisspannung U_{Z} so eingestellt wird, dass die Ausgangsspannungsamplitude des Leistungsoszillators 10 entsprechend der Einstellung des Sollwertgebers 21 geregelt wird.

Der Leistungsoszillator 10 beinhaltet im Allgemeinen, wie in den Figuren 8 und 9 gezeigt, eine Ansteuerschaltung 11 mit Leistungshalbleitern, einen Transformator 12, der durch eine parallel geschaltete Kapazität C_{P} zu einem Parallelschwingkreis ergänzt wird und am Ausgang des Transformators 12 eine Reihenschaltung aus einer Induktivität L_{SA} und einer Kapazität C_{SA}, also einen Serienschwingkreis zum Patientenkreis hin. Bei der in Fig. 9 gezeigten üblichen Ausführungsform ist zusätzlich zwischen den Ausgangsklemmen der Ansteuerschaltung 11 und dem aus dem Transformator 12 und der Parallelkapazität C_{P} gebildeten Parallelschwingkreis ein aus einer Induktivität L_{SE} und einem Kondensator C_{SE} gebildeter weiterer Serienschwingkreis vorgesehen.

Um nun eine möglichst gute Modulierbarkeit und somit ein spontanes Anschwingen der Ausgangsspannung zu gewährleisten, wird oftmals (wie in Fig. 8 gezeigt) auf den Eingangserienschwingkreis (gemäß Fig. 9) verzichtet. Der Eingang des gesamten Schwingkreises ist somit ein Parallelschwingkreis. Da die Ansteuerschaltung wiederum aus einer Spannungsquelle, nämlich einer Ausgangskapazität C_{A} des Netzgerätes 3 gespeist wird, stellt der treibende Leistungshalbleiter 11 einen Kurzschluss zwischen einem geladenen Kondensator (C_{A}) und einem ungeladenen Kondensator (C_{P}) dar. Die Folge hiervon ist, dass die Größe der fließenden Ströme lediglich durch parasitäre Erscheinungen wie Zuleitungsinduktivitäten und Bahnwiderstände der Halbleiter bestimmt wird. Da diese parasitären Werte in aller Regel klein gegenüber den eigentlichen Bauteilwerten sind, entstehen sehr hohe, undefinierte elektrische Stromstärken. Diese oftmals recht hohen Impulsströme können ungewollte elektromagnetische Störungen aussenden (was im Klinikbereich nicht zugelassen wird). Zudem wird die Dimensionierung der Leistungshalbleiter der Ansteuerschaltung 11 oft unwirtschaftlich.

Um diese Problematik zu vermeiden, wird die in Fig. 9 gezeigte Schaltung verwendet, bei welcher die Ansteuerströme durch eine Eingangsinduktivität L_{SE} bestimmt werden. Dies zieht aber den Nachteil nach sich, dass das Filter nicht spontan anschwingt und sich somit für die oben erwähnten Modulationen nicht eignet.

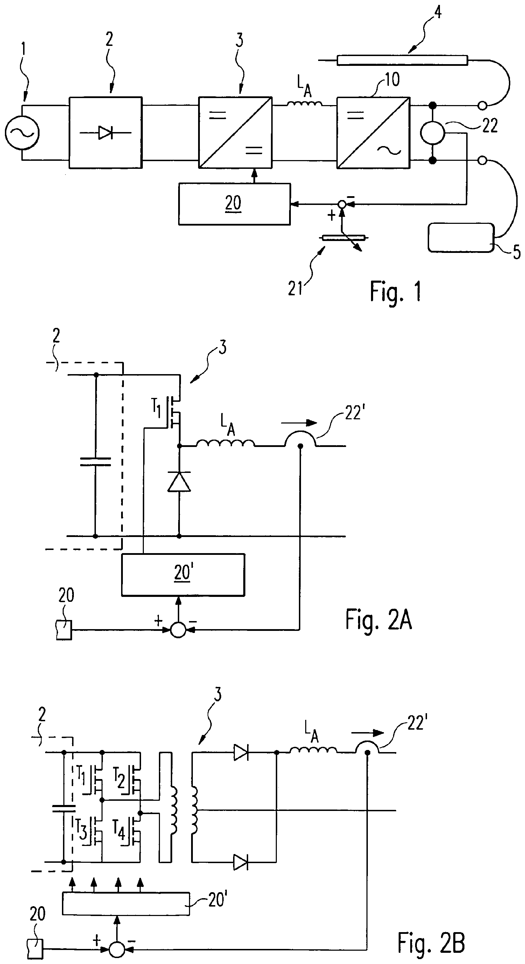

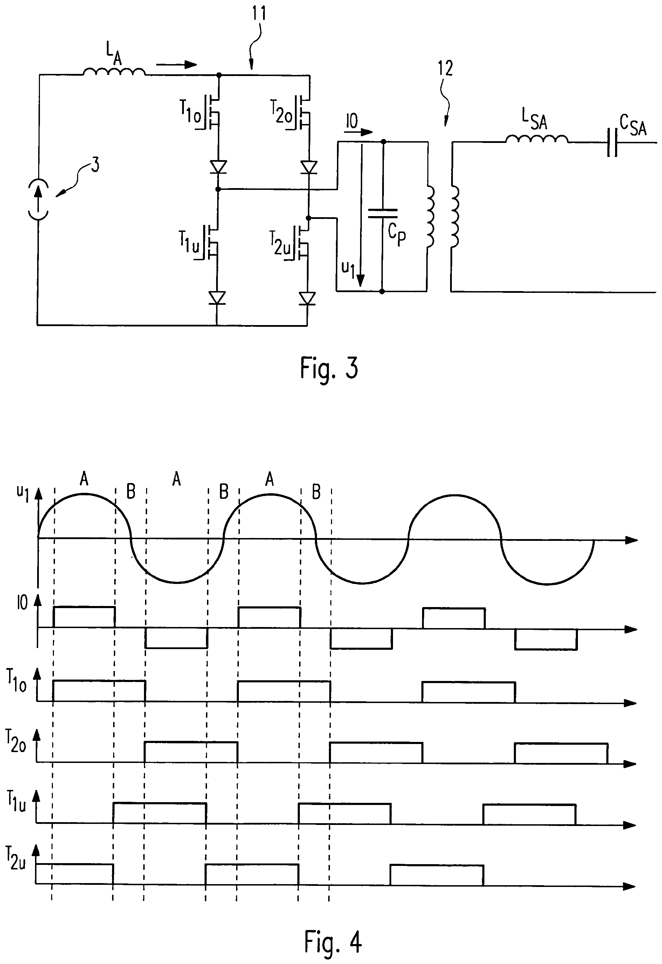

Die Druckschrift US 5,836,943 A befasst sich mit einem HF-Generator für ein elektrochirurgisches Gerät, der gemäß Figur 2 einen Gleichstromregler 10 und einen Leistungsoszillator 100 aufweist. Der Gleichstromregler ist in Figur 3 im Detail dargestellt. Darin ist erkennbar, dass der Gleichstromregler im Wesentlichen als Cuk-Wandler ausgebildet ist, der zu der Gruppe der invertierenden Gleichspannungswandlern gehört. Der Transistor 26 des Cuk-Wandlers wird über einen Regler 70 angesteuert, der über einen Knotenpunkt 38 den Ausgangsstrom des Gleichstromreglers abgreift. Auf diese Weise soll erreicht werden, dass anhand des Ausgangsstroms der Strom des Gleichstromreglers 10 innerhalb eines vorbestimmten Bereichs konstant gehalten werden kann, um einen entsprechenden chirurgischen Effekt hervorzurufen.

Aufgabe der vorliegenden Erfindung ist es, einen HF-Chirurgiegenerator bzw. ein Verfahren zum Erzeugen einer Hochfrequenzspannung in einem HF-Chirurgiegenerator derart aufzuzeigen, dass eine gute Modulierbarkeit erzielt und die genannten Nachteile, insbesondere übergroße Ströme vermieden werden.

Diese Aufgabe wird durch einen HF-Chirurgiegenerator nach Anspruch 1 bzw. ein Verfahren nach Anspruch 10 gelöst.

Insbesondere wird die Aufgabe durch einen HF-Chirurgiegenerator gelöst, der umfasst:
- ein Netzgerät zum Liefern einer gleichgerichteten elektrischen Energie;
- einen Leistungsoszillator zum Liefern einer potentialfreien, gleichstrom- bzw. gleichspannungsfreien Hochfrequenzspannung;
- eine Regeleinrichtung zum Regeln der Hochfrequenzspannung, wobei das Netzgerät als Stromquelle zum Liefern eines eingeprägten Ausgangsstroms ausgebildet ist, dessen Sollwert von der Regeleinrichtung vorgegeben ist, so dass der eingeprägte Ausgangsstrom des Netzgerätes als Stellgröße zum Regeln der HF-Spannung dient.

Ein wesentlicher Punkt liegt also darin, dass - im Unterschied zu den bisher bekannten Lösungen - das Netzgerät keine eingeprägte Ausgangsspannung aufweist, sondern vielmehr einen eingeprägten, geregelten Ausgangsstrom abgibt. Der Sollwert dieses Ausgangsstroms wird vom (an sich bekannten) Regelsystem des HF-Chirurgiegeräts vorgegeben. Der eingeprägte Strom dient somit als Stellgröße für die Regelung der HF-Ausgangsspannung bzw. des HF-Gewebeeffekts.

Vorzugsweise umfasst das Netzgerät eine Netzgleichrichtereinrichtung zum Umwandeln einer Netzwechselspannung in eine im Wesentlichen konstante Gleichspannung und einen nachgeschalteten Zwischenkreis zum Liefern der einstellbaren gleichgerichteten Energie. Hierbei umfasst das Netzgerät vorzugsweise entweder einen Tiefsetzsteller oder einen Potential trennenden Durchflusswandler. Hierbei ist kein Ausgangskondensator vorgesehen. Der Ausgang des Netzgeräts ist somit eine Induktivität und die Regelung des Netzgerätes regelt den Ausgangsstrom, wobei sich die Ausgangsspannung je nach Lastverhältnissen frei einstellen darf.

Da die Regelstrecke der hierbei erforderlichen Stromregelung - zumindest nach geeigneter Wahl der Ausgangsinduktivität, die einen Kompromiss aus Bauvolumen/Kosten und Stromripple darstellt - eine recht schnelle Zeitkonstante aufweist, wird der Stromregler geeigneterweise als ein "Regelsystem mit endlicher Einstellzeit", vorzugsweise sogar als "Dead-Beat-Regler" insbesondere in einem digitalen Signalprozessor oder einem anderen integrierten Schaltkreis realisiert.

In diesem Zusammenhang sei darauf hingewiesen, dass derjenige Zustand des Netzgeräts, in dem keine elektrische Leistung abgegeben wird, der Kurzschluss am Ausgang ist. Das Gerät ist also kurzschlussfest. Im Leerlauf würde die Spannung durch induktive Spannungsüberhöhung bis zur Zerstörung eines Bauteils ansteigen können. Es muss daher durch den Betrieb der nachfolgenden Schaltungsteile (des Leistungsoszillators) sichergestellt werden, dass der induktive Ausgang des Netzteils zu keinem Zeitpunkt im Leerlauf betrieben wird.

Der Leistungsoszillator umfasst vorzugsweise eine als H-Brücke von Halbleiterschaltelementen ausgebildete leistungselektronische Ansteuerschaltung. Zur Vermeidung von ungewollten Lastkurzschlüssen während Freilaufphasen sind vorzugsweise in Reihe mit den Halbleiterschaltelementen Dioden vorgesehen.

Vorzugsweise ist die Ansteuerschaltung derart ausgebildet, dass in einem Rückspeisebetrieb in Blindelementen gespeicherte Energie zum Netzteil rückgespeist wird, wodurch sich der Wirkungsgrad der Anordnung erhöht. Darüber hinaus wird durch die Rückspeisung ein Nachschwingen der Ausgangsspannung vermieden, so dass der Kurvenverlauf der Ausgangsspannung schnellstmöglich auf Null reduziert wird.

Die Ansteuerschaltung ist weiterhin vorzugsweise derart ausgebildet, dass die Halbleiterschaltelemente paarweise phasensynchron in Resonanz angesteuert werden. Durch die relativ hohe Ansteuerzeit von 2 * 50 % ergeben sich somit moderate Stromstärken in den Leistungshalbleitern, die also sehr günstig ausgenützt werden.

Wenn man auf die Rückspeisefähigkeit verzichtet, so können zwei Leistungshalbleiter (gegenüber vier) eingespart werden. Zur Speisung werden entweder zwei unabhängig voneinander geregelte Stromquellen (Netzgeräte) aufgebaut, oder aber die Schaltung wird aus einem stromgeregelten Netzgerät gespeist und die beiden Zweige werden über induktive Stromteiler geteilt.

Das erfindungsgemäße Verfahren zum Erzeugen einer Hochfrequenzspannung in einem HF-Chirurgiegenerator umfasst das Erzeugen einer gleichgerichteten elektrischen Energie in einem Netzgerät, das Erzeugen einer potentialfreien, gleichstrom- bzw. gleichspannungsfreien geregelten Hochfrequenzspannung, wobei das Netzgerät einen eingeprägten Ausgangsstrom mit einem vorgegebenen Sollwert liefert, so dass der eingeprägte Ausgangsstrom des Netzgeräts als Stellgröße die HF-Spannung regelt.

Bevorzugte Ausführungsformen der Erfindung ergeben sich aus den Unteransprüchen sowie aus der nachstehenden Beschreibung bevorzugter Ausführungsformen der Erfindung, die anhand von Abbildungen näher erläutert werden. Hierbei zeigen
- - Fig. 1: eine schematische Darstellung eines HF-Chirurgiegenerators,
- - Fig. 2A: eine erste Ausführungsform eines Netzgerätes für den HF-chirurgischen Generator nach Fig. 1,
- - Fig. 2B: eine zweite Ausführungsform eines Netzgerätes für einen HF-chirurgischen Generator nach Fig. 1,
- - Fig. 3: eine erste Ausführungsform eines Leistungsoszillators für einen HF-chirurgischen Generator nach Fig. 1,
- - Fig. 4: eine Darstellung von Schaltfunktionen von Leistungshalbleitern des Leistungsoszillators nach Fig. 3 zusammen mit der sich dadurch ergebenden Spannung/Strom,
- - Fig. 5: eine zweite Ausführungsform eines Leistungsoszillators,
- - Fig. 6: eine dritte Ausführungsform eines Leistungsoszillators,
- - Fig. 7: eine Darstellung eines bekannten HF-Chirurgiegenerators und
- - Fig. 8 und 9: Ausführungsformen von bekannten Leistungsoszillatoren.

In der nachfolgenden Beschreibung werden für gleiche und gleich wirkende Teile dieselben Bezugsziffern und Bezeichnungen verwendet.

Der prinzipielle Aufbau des hier gezeigten Ausführungsbeispiels des HF-Chirurgiegenerators gemäß Fig. 1 entspricht im Wesentlichen dem nach dem Stand der Technik, wie er anhand der Figuren 7-9 beschrieben wurde. Ein wesentlicher Unterschied liegt jedoch im Netzteil 3, das beim Stand der Technik an seinem Ausgang eine konstante Spannung U_{Z} an einen zum Ausgang parallelen Kondensator C_{A} liefert, während das Netzteil 3 gemäß der Erfindung einen eingeprägten Ausgangsstrom über eine Ausgangsinduktivität L_{A} in den Leistungsoszillator 10 liefert.

In Fig. 2A ist eine erste Ausführungsform eines Netzgerätes 3 gezeigt, das seine konstante Eingangs-Gleichspannung von einem Gleichrichter 2 mit nachgeschaltetem Sieb-Kondensator erhält. Dieses Netzgerät 3 ist als Tiefsetzsteller (allerdings ohne Kondensator am Ausgang) aufgebaut und umfasst eine Reihenschaltung aus einem Schalttransistor T₁ und einer Diode, an deren Koppelpunkt der Leistungsoszillator 10 über die Ausgangsinduktivität L_{A} angekoppelt ist. Der fließende Strom wird über einen Istwertgeber 22' an eine Subtrahierschaltung gegeben, welche den Strom-Istwert mit einem Strom-Sollwert vergleicht, der aus dem oben schon genannten Regler 20 kommt. Der Vergleichswert stellt eine Regelabweichung dar, die einem Regler 20' mitgeteilt wird. Dieser steuert den Transistor T₁ derart, dass sich der gewünschte Ausgangsstrom einstellt.

Die in Fig. 2B gezeigte Variante des Netzgerätes 3 zeigt einen Potential trennenden Durchflusswandler, wie dieser an sich bekannt ist. Es sind hier vier Schalttransistoren T₁, T₂, T₃ und T₄ vorgesehen, die in einer H-Schaltung angeordnet sind. An den Koppelpunkten der Transistoren T₁ und T₃ bzw. T₂ und T₄ ist eine Primärwicklung eines Transformators angeschlossen, dessen miteinander gekoppelte Sekundärwicklungen über Dioden an die Ausgangsinduktivität L_{A} angeschlossen sind. Der Koppelpunkt der Sekundärwicklungen bildet die zweite Ausgangsklemme des Netzgerätes 3. Die Regelung erfolgt entsprechend derjenigen nach Fig. 2A.

Nachfolgend wird die leistungselektronische Ansteuerschaltung für den Leistungsoszillator anhand von Fig. 3 und Fig. 4 erläutert.

Der Leistungsoszillator 10 ist als Γ-Fitter aufgebaut. Seine leistungselektronische Ansteuerschaltung 11 ist gemäß Fig. 3 als H-Brücke mit zwei Paaren von Transistoren T₁ₒ und T₁ᵤ sowie T₂ₒ und T₂ᵤ aufgebaut. Weiterhin sind zur Vermeidung von ungewollten Lastkurzschlüssen während Freilaufphasen Dioden in Reihe zu den Leistungstransistoren vorgesehen. An den Koppelpunkten der Transistorenpaare (T₁ₒ und T₁ᵤ sowie T₂ₒ und T₂ᵤ) ist die Primärwicklung des Transformators 12 mit dem zu ihr parallelen Kondensator C_{P} angeschlossen. Am Ausgang bzw. an der Sekundärwicklung des Transformators 12 ist der Ausgangs-Serienschwingkreis L_{SA} und C_{SA} angeschlossen. Dieser Serienschwingkreis bildet zusammen mit der Sekundärwicklung des Transformators 12 einen Teil des Patientenstromkreises.

Der Betrieb dieser Schaltung gestaltet sich derart, dass - wie in Fig. 4 gezeigt - immer zwei Leistungshalbleiter über Kreuz gleichzeitig leiten. Zum Speisen des Parallelresonanzkreises am Ausgang der H-Schaltung sind also die Transistoren T₁ₒ und T₂ᵤ oder T₂ₒ und T₁ᵤ gleichzeitig leitend. Beim Freilaufzustand, an dem keine Energie vom Netzgerät an den Leistungsoszillator abgegeben wird, ist das gleichzeitige Einschalten entweder von T₁ₒ und T₁ᵤ oder T₂ₒ und T₂ᵤ, also jeweils einer Halbbrücke, notwendig.

Eine dritte erlaubte Betriebsart ist das gleichzeitige Einschalten aller vier Transistoren, womit man einen Rückspeisebetrieb der in den Blindelementen des Leistungsoszillators 10 befindlichen Energie auf die Ausgangsinduktivität L_{A} des Netzteils 3 bewerkstelligt. Mit dieser Art der Rückspeisung lässt sich das Nachschwingen der Ausgangsspannung vermeiden, der Kurvenverlauf der Ausgangsspannung wird also schnellstmöglich auf Null reduziert.

Für die Dimensionierung weist die Schaltung weitere interessante Eigenschaften auf. Es ist nämlich möglich, die Schaltung in einer relativen Ansteuerzeit (Duty-Cycle) von nahezu 100 % anzusteuern. Dabei werden die Schalter T₁ₒ und T₂ᵤ so lange geschlossen, wie die Spannung über dem Parallelschwingkreis (12/C_{P}) positiv ist. Somit erfolgt eine phasensynchrone Ansteuerung, welche die Schaltung in Resonanz betreibt, wie dies aus Fig. 4 hervorgeht. Durch diese große relative Ansteuerzeit von 2 * 50 % ergeben sich moderate Stromstärken in den Leistungshalbleitern, die somit recht günstig ausgenutzt werden.

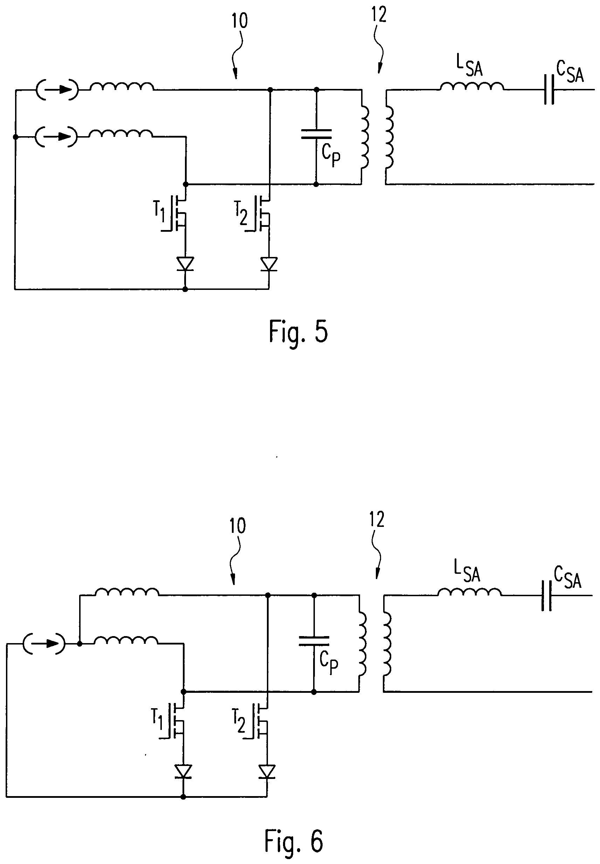

Bei der in Fig. 5 gezeigten Ausführungsform des Leistungsoszillators 10 sind nur zwei Leistungshalbleiter T₁ und T₂ (mit Freilaufdioden) vorgesehen, welche zur Speisung zwei unabhängig voneinander geregelte Stromquellen (Netzgeräte) aufweisen. Bei der in Fig. 6 gezeigten Variante ist nur ein einziges stromgeregeltes Netzgerät vorgesehen, wobei die beiden Zweige mit den darin befindlichen Leistungshalbleitern T₁ und T₂ über einen induktiven Stromteiler gespeist werden.

Aus Obigem geht hervor, dass die Erfindung schaltungstechnisch auf viele verschiedene Weisen realisiert werden kann.

### Bezugszeichenliste

- 1: Netz
- 2: Gleichrichter
- 3: Netzgerät bzw. DC/DC-Wandler
- 4: Instrument
- 5: Neutralelektrode
- 10: Leistungsoszillator
- 11: Ansteuerschaltung
- 12: Transformator
- 20: Regler
- 21: Sollwertgeber
- 22: Istwertgeber

## Patentansprüche

1. HF-Chirurgiegenerator, umfassend
- ein Netzgerät (3) zum Liefern einer gleichgerichteten elektrischen Energie;
- einen Leistungsoszillator (10) zum Liefern einer potentialfreien, gleichstrom- bzw. gleichspannungsfreien Hochfrequenzspannung (U_{HF}); und
- eine Regeleinrichtung (20) zum Regeln der Hochfrequenzspannung (U_{HF});
wobei das Netzgerät (3) als Stromquelle zum Liefern eines eingeprägten Ausgangsstroms ausgebildet ist, dessen Sollwert von der Regeleinrichtung (20) so vorgegeben ist, dass der eingeprägte Ausgangsstrom des Netzgerätes (3) als Stellgröße zum Regeln der Hochfrequenzspannung (U_{HF}) dient, **dadurch gekennzeichnet, dass** das Netzgerät (3) keinen Ausgangskondensator aufweist.

2. HF-Chirurgiegenerator nach Anspruch 1,
**dadurch gekennzeichnet, dass**
das Netzgerät (3) eine Netzgleichrichtereinrichtung (2) zum Umwandeln einer Netzwechselspannung in eine im Wesentlichen konstante Gleichspannung und einen Zwischenkreis zum Liefern der einstellbaren gleichgerichteten Energie umfasst.

3. HF-Chirurgiegenerator nach einem der vorhergehenden Ansprüche,
**dadurch gekennzeichnet, dass**
das Netzgerät (3) einen Tiefsetzsteller umfasst.

4. HF-Chirurgiegenerator nach einem der Ansprüche 1 oder 2,
**dadurch gekennzeichnet, dass**
das Netzgerät (3) einen Potential trennenden Durchflusswandler umfasst.

5. HF-Chirurgiegenerator nach einem der vorhergehenden Ansprüche,
**dadurch gekennzeichnet, dass**
die Regeleinrichtung (20) ein Regelsystem mit endlicher Einstellzeit, insbesondere einen Dead-Beat-Regler umfasst.

6. HF-Chirurgiegenerator nach einem der vorhergehenden Ansprüche,
**dadurch gekennzeichnet, dass**
der Leistungsoszillator (10) eine als H-Brücke (T₁ₒ, T₁ᵤ und T₂ₒ, T₂ᵤ) ausgebildete leistungselektronische Ansteuerschaltung (11) umfasst.

7. HF-Chirurgiegenerator nach einem der vorhergehenden Ansprüche, insbesondere nach Anspruch 6,
**dadurch gekennzeichnet, dass**
in Reihe mit den Halbleiter-Schaltelementen Dioden vorgesehen sind.

8. HF-Chirurgiegenerator nach einem der vorhergehenden Ansprüche, insbesondere nach Anspruch 6,
**dadurch gekennzeichnet, dass**
die Ansteuerschaltung derart ausgebildet ist, dass in einem Rückspeisebetrieb in Blindelementen gespeicherte Energie zum Netzteil (3) rückgespeist wird.

9. HF-Chirurgiegenerator nach einem der vorhergehenden Ansprüche, insbesondere nach Anspruch 6,
**dadurch gekennzeichnet, dass**
die Ansteuerschaltung derart ausgebildet ist, dass die Halbleiterschaltelemente (T₁ₒ, T₁ᵤ und T₂ₒ, T₂ᵤ) paarweise phasensynchron in Resonanz angesteuert werden.

10. Verfahren zum Erzeugen einer Hochfrequenzspannung in einem HF-Chirurgiegenerator nach einem der Ansprüche 1-9, umfassend
- Erzeugen der gleichgerichteten elektrischen Energie in dem Netzgerät,
- Erzeugen der potentialfreien, gleichstrom- bzw. gleichspannungsfreien geregelten Hochfrequenzspannung (U_{HF})
wobei das Netzgerät den eingeprägten Ausgangsstrom mit einem vorgegebenen Sollwert liefert, so dass der eingeprägte Ausgangsstrom des Netzgerätes als Stellgröße die Hochfrequenzspannung (U_{HF}) regelt, wobei sich die Ausgangsspannung je nach Lastverhältnissen frei einstellt.

## Claims

1. RF surgical generator, comprising
- a power supply unit (3) for supplying a rectified electrical energy;
- a power oscillator (10) for supplying a potential-free, direct-current- or DC-voltage-free radio-frequency voltage (U_{RF}); and
- a control device (20) for controlling the radio-frequency voltage (U_{RF});
wherein
the power supply unit (3) is embodied as a current source for supplying an impressed output current, the setpoint value of which is predefined by the control device (20) such that the impressed output current of the power supply unit (3) serves as a manipulated variable for controlling the radio-frequency voltage (U_{RF}),
**characterized in that**
the power supply unit (3) has no output capacitor.

2. RF surgical generator according to Claim 1,
**characterized in that**
the power supply unit (3) comprises a mains rectifier device (2) for converting a mains AC voltage into a substantially constant DC voltage and a link circuit for supplying the adjustable rectified energy.

3. RF surgical generator according to either of the preceding claims,
**characterized in that**
the power supply unit (3) comprises a buck converter.

4. RF surgical generator according to either of Claims 1 and 2,
**characterized in that**
the power supply unit (3) comprises a potential-isolating forward converter.

5. RF surgical generator according to any of the preceding claims,
**characterized in that**
the control device (20) comprises a control system having a finite setting time, in particular a dead-beat controller.

6. RF surgical generator according to any of the preceding claims,
**characterized in that**
the power oscillator (10) comprises a power electronic drive circuit (11) embodied as an H bridge (T₁ₒ, T₁ᵤ and T₂ₒ, T₂ᵤ).

7. RF surgical generator according to any of the preceding claims, in particular according to Claim 6,
**characterized in that**
diodes are provided in series with the semiconductor switching elements.

8. RF surgical generator according to any of the preceding claims, in particular according to Claim 6,
**characterized in that**
the drive circuit is embodied in such a way that energy stored in reactive elements in a feedback mode is fed back to the power supply unit (3).

9. RF surgical generator according to any of the preceding claims, in particular according to Claim 6,
**characterized in that**
the drive circuit is embodied in such a way that the semiconductor switching elements (T₁ₒ, T₁ᵤ and T₂ₒ, T₂ᵤ) are driven in resonance phase-synchronously in pairs.

10. Method for generating a radio-frequency voltage in an RF surgical generator according to any of Claims 1-9, comprising
- generating the rectified electrical energy in the power supply unit,
- generating the potential-free, direct-current- or DC-voltage-free controlled radio-frequency voltage (U_{RF}),
wherein the power supply unit supplies the impressed output current having a predefined setpoint value, such that the impressed output current of the power supply unit as a manipulated variable controls the radio-frequency voltage (U_{RF}), wherein the output voltage is established freely depending on load conditions.

## Revendications

1. Générateur chirurgical HF, comprenant
- un bloc d'alimentation (3) pour fournir une énergie électrique redressée ;
- un oscillateur de puissance (10) pour fournir une tension haute fréquence sans potentiel, sans courant continu ou sans tension continue (U_{HF}) ; et
- un dispositif de régulation (20) pour réguler la tension haute fréquence (U_{HF}) ;
dans lequel le bloc d'alimentation (3) se présente sous la forme d'une source de courant destinée à fournir un courant de sortie appliqué, dont la valeur de consigne est prédéfinie par le dispositif de régulation (20) de sorte que le courant de sortie appliqué du bloc d'alimentation (3) serve de grandeur de réglage pour réguler la tension haute fréquence (U_{HF}),
**caractérisé en ce que** le bloc d'alimentation (3) ne comporte pas de condensateur de sortie.

2. Générateur chirurgical HF selon la revendication 1, **caractérisé en ce que** le bloc d'alimentation (3) comprend un dispositif redresseur de réseau (2) destiné à convertir une tension secteur alternative en une tension continue sensiblement constante et un circuit intermédiaire destiné à fournir l'énergie redressée réglable.

3. Générateur chirurgical HF selon l'une des revendications précédentes, **caractérisé en ce que** le bloc d'alimentation (3) comprend un régulateur abaisseur.

4. Générateur chirurgical HF selon l'une des revendications 1 ou 2, **caractérisé en ce que** le bloc d'alimentation électrique (3) comprend un convertisseur direct à séparation de potentiel.

5. Générateur chirurgical HF selon l'une des revendications précédentes, **caractérisé en ce que** le dispositif de régulation (20) comprend un système de régulation à temps de réponse fini, en particulier un régulateur à réponse continue.

6. Générateur chirurgical HF selon l'une des revendications précédentes, **caractérisé en ce que** l'oscillateur de puissance (10) comprend un circuit de commande électronique de puissance (11) se présentant sous la forme d'un pont en H (T₁ₒ, T₁ᵤ et T₂ₒ, T₂ᵤ).

7. Générateur chirurgical HF selon l'une des revendications précédentes, en particulier selon la revendication 6, **caractérisé en ce que** des diodes sont montées en série avec les éléments de commutation à semi-conducteurs.

8. Générateur chirurgical HF selon l'une des revendications précédentes, en particulier selon la revendication 6, **caractérisé en ce que** le circuit de commande est conçu de sorte que l'énergie emmagasinée dans des éléments factices en mode de réinjection soit réinjectée dans le bloc d'alimentation (3).

9. Générateur chirurgical HF selon l'une des revendications précédentes, en particulier selon la revendication 6, **caractérisé en ce que** le circuit de commande est conçu de sorte que les éléments de commutation à semi-conducteurs (T₁ₒ, T₁ᵤ et T₂ₒ, T₂ᵤ) soient commandés par paires en résonance et en synchronisme de phase.

10. Procédé pour générer une tension haute fréquence dans un générateur chirurgical HF selon l'une des revendications 1-9, consistant à
- générer l'énergie électrique redressée dans le bloc d'alimentation,
- générer une tension haute fréquence régulée sans potentiel, sans courant continu ou sans tension continue (U_{HF}),
dans lequel le bloc d'alimentation fournit le courant de sortie appliqué avec une valeur de consigne prédéterminée de sorte que le courant de sortie appliqué du bloc d'alimentation régule la tension haute fréquence (U_{HF}) en tant que grandeur de réglage, dans lequel la tension de sortie peut être librement réglée en fonction des conditions de charge.
